# EUROPEAN PATENT SPECIFICATION

(11) **EP 1 511 849 B1**
(45) Date of publication and mention of the grant of the patent: **23.08.2006**
(21) Application number: 03730913.5
(22) Date of filing: 30.05.2003
(51) Int. Cl.: C12Q 1/00, C12Q 1/48, G01N 33/68, G01N 35/08

(54) **CONTINUOUS-FLOW ENZYME ASSAY WITH MASS SPECTROMETRY DETECTION**
DURCHFLUSS ENZYMASSAY MIT EINEM MASSENSPEKTROMETRISCHEN NACHWEIS
DOSAGE D'ENZYME A FLUX CONTINU

(30) Priority: 31.05.2002 EP 02077146
(43) Date of publication of application: 09.03.2005
(73) Proprietor: Kiadis B.V., 2333 CA Leiden (NL)
(72) Inventor: IRTH, Hubertus, NL-1078 NP Amsterdam (NL); LETZEL, Thomas, 80634 Munich (DE)
(74) Representative: Winckels, Johannes Hubertus F.
(86) International application number: PCT/NL2003/000408
(87) International publication number: WO 2003/102222

(56) References cited:
- WO-A-02/37111
- INGKANINAN K ET AL: "High-performance liquid chromatography with on-line coupled UV, mass spectrometric and biochemical detection for identification of acetylcholinesterase inhibitors from natural products" JOURNAL OF CHROMATOGRAPHY, ELSEVIER SCIENCE PUBLISHERS B.V. AMSTERDAM, NL, vol. 872, no. 1-2, March 2000 (2000-03), pages 61-73, XP004190228 ISSN: 0021-9673 cited in the application
- LANGRIDGE JAMES I ET AL: "Analysis of cyclic nucleotide-related enzymes by continuous-flow fast-atom bombardment mass spectrometry." RAPID COMMUNICATIONS IN MASS SPECTROMETRY, vol. 7, no. 4, 1993, pages 293-303, XP008010052 ISSN: 0951-4198 cited in the application
- ZHANG BOYAN ET AL: "Frontal affinity chromatography coupled to mass spectrometry for screening mixtures of enzyme inhibitors." ANALYTICAL BIOCHEMISTRY, vol. 299, no. 2, 15 December 2001 (2001-12-15), pages 173-182, XP002219168 ISSN: 0003-2697 cited in the application
- CRAIG A GREY ET AL: "Monitoring protein kinase and phosphatase reactions with matrix-assisted laser desorption/ionization mass spectrometry and capillary zone electrophoresis: Comparison of the detection efficiency of peptide-phosphopeptide mixtures." BIOLOGICAL MASS SPECTROMETRY, vol. 23, no. 8, 1994, pages 519-528, XP008010048 ISSN: 1052-9306
- MURRAY K J ET AL: "USE OF A SYNTHETIC DODECAPEPTIDE (MALANTIDE) TO MEASURE THE CYCLIC AMP-DEPENDENT PROTEIN KINASE ACTIVITY RATIO IN A VARIETY OF TISSUES" BIOCHEMICAL JOURNAL, PORTLAND PRESS, LONDON, GB, vol. 267, 1 May 1990 (1990-05-01), pages 703-708, XP000945069 ISSN: 0264-6021
- KHOURI H E ET AL: "A MODEL TO EXPLAIN THE PH-DEPENDENT SPECIFICITY OF CATHEPSIN B-CATALYZED HYDROLYSES" BIOCHEMICAL JOURNAL, vol. 275, no. 3, 1991, pages 751-758, XP008025017 ISSN: 0264-6021

## Description

The present invention relates to the measurement of enzyme activity and the modification and especially inhibition thereof. Particularly, the invention relates to a continuous-flow enzyme assay. Furthermore, this invention relates to the use of newly detected compounds giving interaction with a particular enzyme.

Enzyme assays are highly sensitive detection techniques that can be used to characterize both enzymes and substrates by the detection of conversion products. Typically, an enzyme and its substrate are mixed together with, *e*.*g*., co-factors at appropriate buffer conditions, and after a certain incubation time, the reaction is stopped and the amount of products formed during the reaction is measured.

The measurement is typically based on the change of detection properties of the substrate. For example, the measurement can be based on the conversion of a non-fluorescent substrate in a reaction product that has fluorescent properties which can be used as signals for fluorescence detection. Similarly, a colorless substrate can be converted into a colored product to be measured by UV-VIS detection.

From the amount of product formed as a function of time, conclusions can be drawn on the kinetics of the enzyme-substrate reaction.

Substances can be tested for enzyme inhibition activity by adding a certain concentration of the said substance to the enzyme-substrate reaction mixture, and detecting a change of product formation in comparison with a measurement where no substance was added.

Enzyme assays can be carried out in various formats. A common format relates to batch assays carried out in a microtitre plate. Enzyme, substrate and substance to be tested for inhibition are pipetted into a well of the microtitre plate and the amount of product formed is measured after a predefined time.

Another approach involves the immobilization of the enzyme on a solid surface, for example of a chromatographic particle. The immobilized enzyme material is then packed into a reactor, and the substrate is pumped through the reactor. Product detection occurs downstream of the solid-phase reactor using a suitable detector. The inhibition activity of substances is tested by adding the substance to the substrate prior to introduction in the enzyme reactor. The decrease of product formation by substances exhibiting enzyme inhibition is measured downstream to the reactor. In this light, reference can be made to several papers describing the use of the so-called immobilized enzyme reactors (IMER) in a continuous-flow system coupled to a mass spectrometer. See in this respect, *e*.*g*., Amankwa *et al*. in Protein Science 4 (1995) 113-125; Jiang *et al*. in J. Chromatogr. A 924 (2001) 315-322; Mao *et al*. in Anal. Chem., 74 (2002) 379-385; and Richter *et al*. in Anal. Chem., 68 (1996) 1701-1705. These IMERs were mainly used to study substrate conversion reactions and the physico-chemical parameters that influence the extend of the enzyme conversion reaction. No reference is made to the study of enzyme inhibitors by IMERs. Although such IMERs could principally be coupled on-line to a fractionation method such as liquid chromatrography, its operational performance would be very disadvantageous since the elution of an irreversible inhibitor could easily block the entire IMER and thereby prevent any further measurements. The user would have to exchange the reactor before continuing the screening of other samples.

In yet another known format, the enzyme reaction is performed in a continuous-flow reaction system. The reaction time in the continuous-flow reaction system is constant and is determined by the volume of the reactor and the total flow rate. Enzyme and substrate are separately pumped and mixed with a carrier stream containing the substance to be tested. In the absence of inhibition activity, a maximum amount of product is formed during the available, constant reaction time. The presence of an inhibitor leads to a decrease of product formation which can be detected by a suitable detector downstream to the continuous-flow reaction system An example of such a continuous-flow assay described in the state of the art is given by Ingkaninan *et al*. in J. Chromatogr. A, 872 (2000), 61-73. They describe the set up of a specific continuous-flow enzyme assay using UV-VIS detection as a readout. Particularly, they describe that acetylcholine-esterase and its substrate were mixed in a continuous-flow reaction detection system, and the presence of inhibitors in the carrier fluid was detected by measuring a colored product of the enzyme substrate reaction at 405 nm.

For this type of continuous-flow assay, there are a number of principal drawbacks of which a number will be discussed in the following paragraphs.

First, the method of Ingkaninan *et al*. relies on particular requirements of the substrate; that is, a suitable substrate has to be found that can be converted into a product suitable for UV-VIS detection. Although a number of such substrates are known in the art in association with various enzyme systems, there is a very large group of enzymes, and particularly pharmaceutically relevant enzymes, such as kinases, which lack a suitable substrate working according to the detection principle described by Ingkaninan *et al*.. This problem can, in principle, be overcome by using a more complicated assay protocol, e.g., a protocol wherein the use of labeled antibodies against products formed in the enzyme substrate reaction would have to be employed. However, this makes assay development and the possibility of using the assay on-line with a fractionation step very difficult.

Corresponding disadvantages occur for other detection methods which rely on a difference in signal between substrate and converted substrate. A well-known example is detection by means of fluorescence.

Moreover, UV-VIS is a rather insensitive detection method. Accordingly, assays of the type described by Ingkaninan *et al*. are characterized by a relatively low detection sensitivity.

In addition, it is noted that the assay format described by Ingkaninan *et al*. is described as a method for the assaying of a single enzyme.

It is a primary aim underlying the present invention to provide a method or assay which overcomes at least one of the drawbacks of the existing batch and continuous-flow assays. Particularly, it is aimed to make the assay or method of the invention more flexible as compared to the enzyme assays based on fluorescence or UV-VIS detection, especially in view of the disadvantage associated with the sketched requirement of a suitable substrate. Substrates used for fluorescence or UV-VIS detection are typically chemical derivatives of native enzyme substrates, where the original substrate is covalently linked to a chemical moiety that can be detected - either directly or after enzyme cleavage - by the respective detection methods. The synthesis of detectable substrates often leads to prolonged assay development times since chemical alteration of the original substrate often leads to decreased substrate specificity and consequently to very insensitive assays.

Moreover, the assay format of the present invention should be capable of being linked to a fractionation method such as liquid chromatography.

Further, it would be desirable to have available a method for assaying multiple enzyme combinations.

The present invention solves any or more of the problems of the known enzyme activity measurements assays and/or meets the desires mentioned above by making use of the detection of conversion products of an enzyme-substrate reaction by means of mass spectrometry.

By using mass spectrometry as the detection technique, substrate conversion products can be detected directly; no chemical derivatization of the native substrate is required for assay development.

In addition, the present invention makes it possible both to study enzyme kinetics and to screen for active ligands inhibiting or activating the enzyme or otherwise modifying the enzyme activity.

In a particular embodiment the present method is used to screen enzymes with hitherto unknown substrate activities.

The present invention relates to a continuous-flow enzyme reaction system where one or more of the conversion products are detected on-line by mass spectrometry, such as electrospray mass spectrometry. The continuous-flow enzyme reaction system readout can be generated in such a way that it can be coupled to separation methods such as liquid chromatography or capillary electrophoresis in order to screen mixtures of compounds.

More in detail, the present invention relates to an on-line detection method comprising the steps of: contacting an effluent of a fractionation step with a controlled amount of an enzyme; allowing the enzyme to interact with compounds capable of binding to or intimately interacting with said enzyme so that the enzyme properties are affected, said compounds being suspected to be present in the effluent; addition of a controlled, amount of a substrate for said enzyme; allowing a reaction of the enzyme with the substrate providing one or more modified substrate products; and detection of unreacted substrate, or a modified substrate product using a mass spectrometer.

It is to be noted that several methods describing the use of mass spectrometry in respect to enzyme detection have been described in the state of the art. The presently claimed method is however not disclosed nor suggested.

For instance, reference can be made to an article of Ge *et al*. in Anal. Chem., 73 (2001) 5078-5082. Ge *et al*. propose the use of mass spectrometry for the determination of enzyme kinetics. Particularly, the incubation of the enzyme glutathione S-transferase with glutathione and 1-chloro-2,4-dinitrobenzene is described, wherein after certain time intervals the amount of product formed in the enzyme reaction is measured by electrospray ionization mass spectrometry. This known method allows the monitoring of an enzyme reaction by using the enzyme's original or natural substrate. Ge *et al*. only teach batch applications, i.e., incubation of sample, enzyme and substrate, and subsequent injection of the reaction product into the mass spectrometer. This method is therefore only useful for the determination of enyzme kinetics and the screening of pure substances. The method of Ge *et al*. can not be coupled on-line to a fractionation step, and the screening of mixtures as made possible by the present invention can only be performed by off-line fraction collection after fractionation of the sample using, for example, liquid chromatography.

A similar method is proposed by Norris *et al*. (Anal. Chem., **73** (2001), 6024-6029) who use batch incubations to study the kinetic characterization of certain inhibitors of fucosyltransferase.

Also in the above-identified article of Ingkaninan *et al.* an embodiment is described wherein the on-line system using UV spectrometry additionally makes use of a mass spectrometry unit to identify particular components found by the said on-line system. That is, if on the basis of the UV measurement activity is noted, electro-spray ionisation spectrometry can be used to obtain information for the molecular mass of the active compounds. Any suggestion to use the mass spectrometer as the apparatus to determine whether there is an interaction between an unknown compound and the enzyme to be screened, as in the method of the present invention, is absent.

Langridge *et al*. in Rapid Comm. Mass Spectrometry, 7 (1993), 293-303 teach the analysis of particular enzymes using continuous flow Fast Atom Bombardment Mass Spectrometry. The article focuses on the establishment of the techniques and its sensitivity and reproducibility. Any reference to the coupling to a fractionation step is absent; enzyme reactions are monitored on line.

Boyan *et al*. describe in Anal. Biochem., **299** (2001) at pages 173-182 a screening of mixtures of enzyme inhibitors by frontal affinity chromatography coupled to MS. This article aims to investigate enzyme substrate kinetic parameters and binding constants. Thereto a substrate measurement is carried out to determine the quality of the enzyme after immobilisation to an affinity column. No continuous flow processes are described.

In summary, all prior art methods described herein-above wherein enzyme assays are performed in combination with mass spectrometry as readout technique are either carried out in batch or in association with immobilized enzyme reactors (IMER). Both approaches can be used mainly for the investigation of enzyme kinetics and for the screening of single, pure compounds. Contrary to the method of the present invention, these methods cannot be coupled directly to a separation method such as liquid chromatography or capillary electrophoresis since incubation and detection are discontinuous processes. Application of mixtures to both types of enzyme assay formats does not reveal the nature of the compound in the mixtures that has caused the inhibition activity. In addition, the method of the inventions does not require the implementation of synthesized fluorescent or radioactive labels for the detection of bioactive compounds.

The present invention is based on the combination of continuous-flow enzyme assays with mass spectrometry as readout technique. In a first step of this on-line detection method an effluent of a fractionation step is contacted with a controlled amount of at least one enzyme. A suitable fractionation method to be used in the methods of the present invention comprises a liquid chromatography separation or a capillary electrophoresis step. Other separation or fractionation techniques which are known to the person skilled in the art and which allow a relatively continuous output stream can, however, be used as well.

Not only can the fractionation step be a liquid chromatography separation, a capillary electrophoresis step, but also a combinatorial chemistry system.

Preferred liquid chromatography fractionation steps comprise HPLC, reversed phase HPLC, capillary electrophoresis (CE), capillary electrochromatography (CEC), isoelectric focusing (IEF) or micellar electrokinetic chromatography (MEKC), all of which techniques are known to the person skilled in the art. In a preferred embodiment, the liquid chromatography separation step is a reversed phase HPLC step.

It will be understood that according to the present invention as the effluent of a fractionation step is also to be understood the effluent of a flow injection analysis system, in which a mixture of different compounds is injected. Since the flow injection technique provides a flexible and fast screening method, it is a preferred embodiment of the present invention.

In the next step, the enzyme is allowed to interact with compounds capable of binding to or otherwise intimately interacting with the enzyme so that the enzyme properties are affected which compounds are suspected to be present in the effluent. In this description and the appending claims, the said compounds are also referred to "analyte". The term "analyte" comprises both known or new substrates for an enzyme, but also enzyme inhibitors or enzyme modifiers. To effect that the detection method can be used in a real-time manner, the duration of the interaction is rather short, with a maximum of about 5 and preferably about 3 minutes. The minimum interaction time depends somewhat on the enzyme used but generally is at least 30 seconds and preferably at least 1 minute. Good results were obtained by the present inventors with interaction times of 2-3 minutes.

In a next step, a controlled amount of a known substrate for the enzyme(s) used is added and said amount is allowed to react with the enzyme providing one or more modified substrate product. Finally, the unreacted substrate, or one of the modified substrate products are read-out using a mass spectrometer.

Preferably, the method of the present invention involves detection of a modified substrate product.

The entire reaction mixture can be introduced into a mass spectrometer without prior separation of substrate and the modified substrate products. The change of amount of known compound to be detected by the MS indicates the presence of at least one analyte in the effluent giving interaction with the enzyme.

In the preferred embodiment wherein a modified, and in general a relatively small, substrate product is used in the MS detection step, preferably a hollow-fibre module is inserted prior to the mass spectrometer in order to remove the high molecular mass fraction of the reaction mixture to be analysed. Particularly, the reaction mixture passes a hollow-fibre module, separating off molecules which have a higher molecular weight said modified substrate product, prior to entering the mass spectrometer. More preferably, the hollow-fibre module splits off all compounds having a size larger than the modified substrate product, inclusive of the substrate used. Of course, also other apparatuses capable of splitting off larger molecules can be used.

In a preferred embodiment of the method of the invention, a make-up flow is added to the reaction mixture resulting from the reaction with the substrate, prior to the introduction in the mass spectrometer. A make-up flow contains ingredients that improve the detection sensitivity of the products formed in the enzyme substrate reaction. Examples of such ingredients are organic solvents such as methanol and acetonitrile, as well as acids like acetic acid and formic acid. These solvents and acids interact with the compounds to be detected and make that such compounds are for instance easier ionised.

The required selectivity can be obtained by operating the MS such, that selectively tracing of the MS is obtained.

All modes of MS-operation are possible in Flow Injection mode, however, due to the higher background, especially the very selective modes which comprise detecting ions of a selected single m/z trace or of selected multiple m/z traces are suitable.

Preferably, the MS is of the type chosen from the group consisting of electrospray ionisation type, atmospheric pressure ionisation type, quadrupole type, magnetic sector type, time-off-flight type, MS/MS, MSⁿ, FTMS type, ion trap type and combinations thereof. A very suitable method, illustrated in example 3 is electron spray time-of-flight mass spectrometry (ESI-TOF-MS).

For example, in scanning mode to trace compounds, low resolution MS with all possible instrumental designs of MS can be used, in particular quadrupole, magnetic sector, time-off-flight, FTMS and ion-trap. This generates typically molecular weight data with nominal mass accuracy.

When high resolution MS is applied, using all possible high resolution instrumental designs, in particular magnetic sector, time-off-flight, FTMS and ion trap, molecular weight data with high mass accuracy combined with the elemental composition of the compound can be obtained.

Other known MS techniques comprise tandem MS, such as MS/MS or MSⁿ (for example MS³). Application of these techniques enables the collection of structural information of the ligands which is a preferred embodiment of the present invention. The data in scanning mode can be acquired in data-dependent mode which means that for each peak observed automatically the tandem MS measurement is performed. In addition to this the fragmentation induced in the tandem MS measurement can be coupled by more sophisticated procedures, for example a broad band excitation, which also fragments expected fragments of less importance, such as the [protonated molecule-H2O]⁺ peak in natural product screening.

The MS can also be operated in single/multiple ion monitoring mode, which can be used for highly selective detection.

Single/multiple ion monitoring mode can be used for highly selective detection. Using low resolution MS in single/multiple ion monitoring mode with all possible low resolution instrumental designs, in particular quadrupole; magnetic sector, time-off-flight, FTMS and ion trap, selective detection based on monitoring of previously determined m/z trace can be obtained. When high resolution MS is applied all possible instrumental designs of MS can be used, especially quadrupole, magnetic sensor, time-off-flight, FTMS and ion-trap. This enables typically very selective detection based on monitoring in high resolution previously determined m/z trace. When tandem MS is applied, all possible instrumental designs, in particular ion trap, quadrupole-time-off-flight (Q-TOF), triple quadrupoles (QQQ), FTMS and combinations of sector instruments with quadrupoles and ion traps, can be used. This enables the very selective detection based on monitoring a resulting peak or peaks in MS/MS and/or MSⁿ measurements.

It is also possible to operate the MS in scanning mode. In this way the lowering of the pre-determined signal is correlated to the increase of other signals, enabling the active compound to be characterized in the same cycle.

With the assays according to the present invention the tracing of (bio-) active compounds, interacting with a particular enzyme, in solutions of complex nature, for instance biological fluids or extracts, natural product extracts, solutions or extracts from biotechnological processes, resulting from chemical experiments, such as combinatorial technologies and processes and the like can be performed with a higher efficiency, selectivity and flexibility. Moreover, the present invention provides the possibility to limit the disturbance of background compounds.

The present invention further enables the identification of compounds based on conventional mass spectra, high resolution data or MSⁿ based spectra.

With the method of the present invention it is also possible to perform library searching, based on a variety of mass spectrometric experiments enabling the screening of large series of samples and classifying these in classes based on similar active compounds, without the need for full identification of the compounds.

The assay method of the present invention can be applied in miniaturized formats of assay-based systems, for instance with chip technology based screening systems.

Contrary to the known methods described herein-above, the method of the invention makes it possible to use a mixture of two or more different types of enzymes. In this preferred embodiment also a mixture of different substrates should be used, each of these substrates being specific for one of said enzymes. The presence of an active compound giving interaction with one of the enzymes in a mixture can by monitoring the changes of the concentration of a known substrate or modified substrate product at its specific mass to charge ratio. Moreover, the molecular mass of the active compound can be measured simultaneously by monitoring the total ion current chromatogram at the time where the peak maximum for the known ligand occurs.

In another preferred embodiment, which is also elaborated in the working examples, the method of the invention uses as the enzyme or one of the enzymes a protein kinase. In that embodiment very good results are obtained when the detection is carried out on a phosphorylated product of a kinase catalysed reaction. The detection can well be used to show the presence of enzyme inhibitors.

In another aspect, the present invention relates to an on-line detection method, wherein a mass spectrometer with a multiple-inlet unit is used, to which multiple-inlet unit different fractionation lines are connected, wherein each fractionation line comprises an effluent to which controlled amounts of enzyme and known substrates are added as described in each of the embodiments of the method of the present invention as described in the present description.

Also, the invention relates to new compounds detected by the method of the present invention. Such compounds have the possibility of giving interaction with the enzyme added in the assay method.

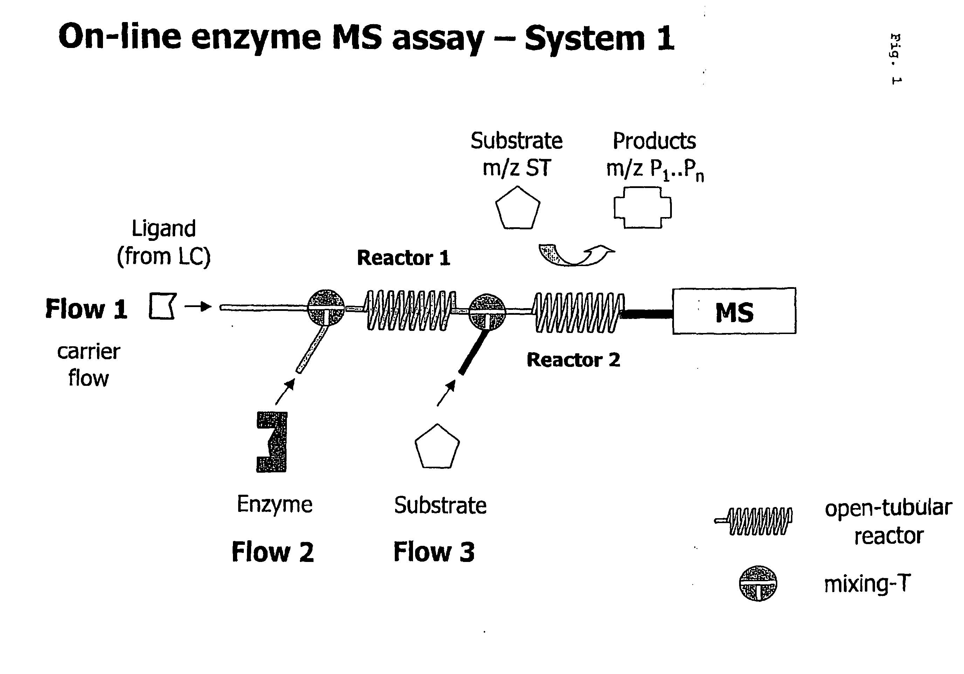

The method of the present invention will be described in more detail, while referring to the embodiments described in the figures 1-3, which schematically show three non-limiting embodiments of the method of the present invention. Particularly, the apparatuses used in the method of the present invention which are described in some detail for each of the 3 embodiments shown in the figures, can, in general, also be used in the embodiments described in the other figures.

In figures 1-3, the reaction between enzyme and substrate proceeds in a continuous-flow reaction detection system comprised of an open-tubular, knitted reactor 1. In order to achieve maximum sensitivity, the assay is performed in a sequential mode. In a first step, the carrier stream Flow 1 containing the compounds to be tested is mixed with the enzyme solution Flow 2. The carrier stream can for instance be the carrier of a flow injection analysis (FIA) system or the outlet of a continuous-flow separation technique such as HPLC or CE. The volume of Reactor 1 and the total flow rate of liquid streams Flow 1 and Flow 2 determine the incubation time. The incubation time can easily be varied by changing the reactor volume or the flow rates and thus gaining insight in the kinetics of the enzyme inhibition.

In a second step, the substrate Flow 3 is mixed with the liquid stream emanating from Reactor 1, initiating the substrate conversion reaction. The volume of tubular Reactor 2 and the total flow rate of liquid streams Flows 1, 2 and 3 determine the substrate incubation time. Again, the incubation time can easily be varied by changing the reactor volume or the flow rates and thus gaining insight in the kinetics of the enzyme substrate reaction.

The outlet of Reactor 2 can be connected to for instance an electrospray interface of the mass spectrometer in three different ways, i.e., (i) directly without further modification of the reaction mixture (Figure 1), (ii) directly after addition of a make-up flow consisting typically of methanol in combination with acetic or formic acid and an internal standard (Figure 2) and (iii) via a hollow-fibre interface (Figure 3). In the latter case only the permeate of the hollow-fibre module containing the low molecular mass reaction products is directed towards the mass spectrometers, whereas the high-molecular mass fraction in the retentate of the hollow-fibre is directed to waste.

In Figure 1, the reaction products (P₁..Pₙ) of the enzyme reaction are measured directly at their molecular mass. The enzyme reaction is carried out in a MS compatible, volatile buffer consisting, for example, of ammonium acetate, ammonium formate or ammonium carbonate. In the absence of inhibiting substances, a constant amount of product is formed in the continuous-flow reaction system due to the well-defined, constant reaction time of the enzyme-substrate reaction. The formation of a constant product concentration leads to a constant MS signal at the specific m/z value of the products-that are selected as indicators for the extent of the enzyme substrate reaction. The injection of an inhibitor (or another compound influencing the enzyme-substrate interaction) into the carrier flow or the elution of an inhibitor from a separation method leads to a decrease of the enzyme activity (reaction in Reactor 1) and, consequently, to a decrease of the product concentration formed in Reactor 2. The addition of an internal standard, typically a low molecular mass organic compound with similar chemical characteristics of the enzyme reaction product, to the substrate solution is used to control the constancy of the electrospray interface conditions. A decrease of the product signal can only be considered as specific and caused by an inhibitor if the signal for the internal standards remains constant during the measurement period. If both product and internal standard signals are decreasing simultaneously, the signal is probably caused by a change of ionization conditions, for example due to matrix components, and not due to the specific inhibition of the enzyme.

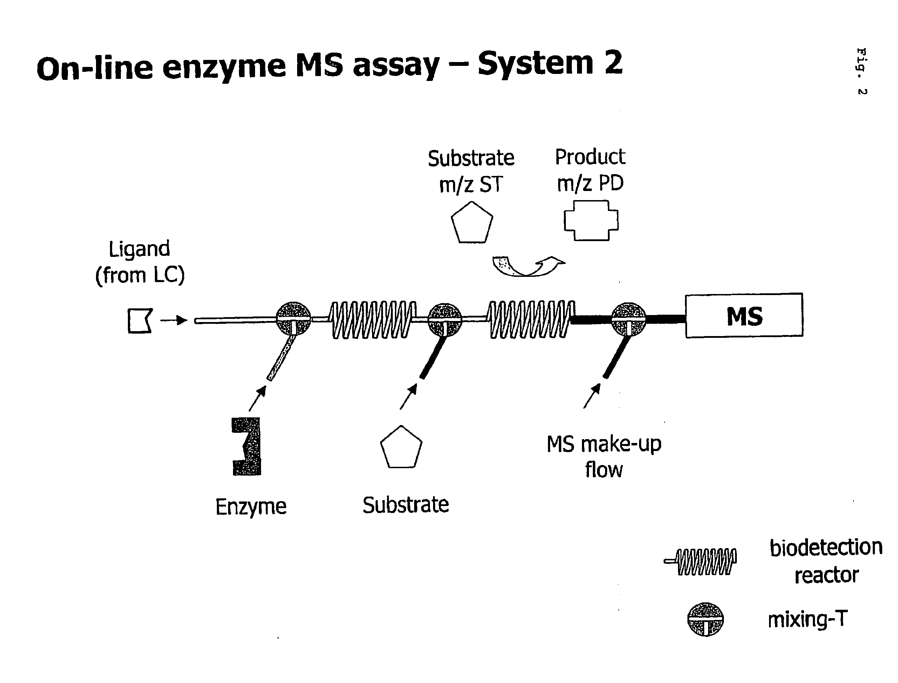

Figure 2 is identical to Figure 1 except that, prior to the introduction of the reaction product into the mass spectrometer, a make-up flow is added containing an organic solvent such as methanol or acetonitrile, a volatile organic acid such formic acid or acetic acid and an internal standard. The scheme shown in Figure 2 is used in those cases wherein the enzyme reaction products serving as an indicator for the extent of the enzyme-substrate reaction exhibit a weak ionisation efficiency and, consequently, can only be measured at relatively high concentration. It is well known that the addition of an organic solvent and a volatile acid results in an improved detection sensitivity of organic molecules in electrospray mass spectrometry. Similarly to the method shown in Figure 1, the internal standard is used to check the stability of the electrospray ionisation conditions.

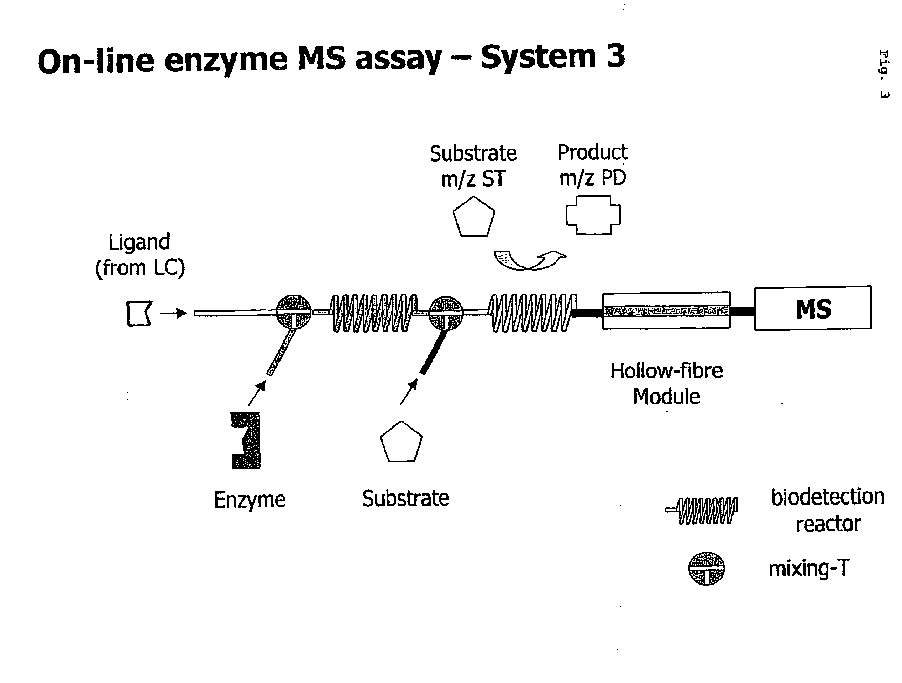

The method sketched in Figure 3 is applied in those cases where the presence of a high molecular fraction such as the enzyme itself or protein impurities from the production of the enzyme causes a large amount of ion suppression. By introducing the reaction product into a hollow-fibre module, the high molecular mass fraction is removed, and only the permeate containing the low molecular mass reaction products is directed towards a mass spectrometer. Similar to the embodiment shown in Figure 2, a make-up flow can be added to the permeate prior to introduction into the MS in order to enhance the ionisation efficiency and, thus, the detection sensitivity of the reaction products.

Since the substances to be tested or screened, which are injected either directly into the carrier flow or eluate on-line from a suitable fractionation technique, are directed to the MS together with the reaction products of the enzyme substrate reaction, the method of the present invention is able to simultaneously determine the biochemical properties of this substance, i.e., its potential to inhibit the enzyme, and its molecular mass. The latter information can be used to elucidate the structure of the substance to be tested.

The enzyme assay method of the present invention can be coupled on-line to a suitable fractionation technique since the enzyme is added continuously to the carrier stream. This overcomes the disadvantage of methods described as prior art herein-above, where the enzyme is either added to batch solutions of the sample or immobilized on a solid-phase material.

In case of batch incubations, the reaction time has to be carefully controlled since otherwise no comparison between different samples would be possible. Moreover, batch incubations cannot be coupled on-line to fractionation techniques, but require an off-line fraction collection of the eluate of the fractionation technique.

The major drawback of using immobilized enzyme reactors lies in the possibility that an active compound or a matrix constituent inactivates the immobilized enzyme and requires the introduction of a fresh IMER column. Since a possible deactivation cannot be predicted, methods using IMERs require frequent control measurement to test the activity of the IMER. In the method of the present invention, fresh enzyme proteins are continuously added at a low flow rate to the carrier flow. The injection of an active inhibitor or an interfering matrix component therefore leads only to a temporary deactivation of the enzyme.

The methods described in the prior art discussed herein-above cannot be coupled on-line to a fractionation and are therefore not suitable for the screening of mixtures of compounds.

By using mass spectrometry as detection method for the products of the enzyme substrate reaction the present invention has several advantages in comparison with on-line enzyme assays using ITV-VIS detection as described by Ingkaninan *et al*.. Most importantly, assay development does not require the chemical (covalent) attachment of a detectable moiety to an existing substrate. It is well known that such a chemical alteration may lead to an inactivation of the substrate. In the present invention the use of the native substrate for the enzyme is possible. During assay development the specific MS detection conditions for the products of the enzyme substrate reaction are established and optimized. These conditions are subsequently used in the screening of enzyme inhibitors.

Another advantage over assays based on UV-VIS detection as described by Ingkaninan *et al.* is the potential of the present invention to screen several enzymes simultaneously. In this case, mixtures of the enzymes and their specific substrates are used rather than single components. The mass spectrometer detects the individual reaction products for each enzyme assay simultaneously at their specific m/z value. These multi-enzyme assays can be performed without designing specifically labeled substrates that can be distinguished on basis of, for example, their absorption wavelength in UV-VIS detection or their excitation and emission wavelength in fluorescence detection. From the total ion current data generated by the mass spectrometer, those mass traces are isolated for interpretation of the individual enzyme assays that represent the specific reaction products.

The on-line coupling to a fractionation technique therefore allows, unlike batch assays or assays performed using immobilized enzyme reactors, the screening of mixtures of compounds. A preferred method of screening of mixtures consist of the following steps. The mixture, for example a natural product extract, a reaction product from combinatorial chemistry, or a reaction product from a metabolic profiling experiment, is injected into a suitable fractionation technique, for example, an HPLC separation column. Compounds eluting from the HPLC column are mixed with the enzyme by adding the enzyme in a mixing unit. In Reactor 1, compounds eluting from the HPLC column are allowed to interact with the enzyme for a well-defined amount of time determined by the flow rate of the HPLC and enzyme flow streams. Subsequently, via a second mixing unit, the substrate is added and after a second reaction time determined by the volume of Reactor 2 and the total flow rates of the HPLC, the enzyme and the substrate flow streams, the mixtures is directed towards a mass spectrometer using for instance the methods shown in Figures 1-3. The elution of an active compound from the HPLC column leads to a change in the behaviour of the enzyme, such as a partial or full deactivation of the enzyme, depending on its inhibition constant. The inhibition of enzyme molecules brought into contact with the active substance leads to a decreased product formation in Reactor 2 which is detected by, e.g., electrospray MS. After the active substance has left Reactor 2, the signal retains its original value. The presence of an active compound leads to a negative peak in the mass trace of the enzyme reaction products.

A comparison of the MS data obtained when analytes are injected with the signal obtained when only the controlled amounts of substrate or modified substrate products are introduced in the continuous-flow system, provides information in respect of the percentage of the interaction with enzyme by the analytes present in the effluent of the fractionation step. The person skilled in the art possesses the knowledge to process and evaluate the data obtained from the detection method. The percentage interaction by an analyte present in the effluent can be used to find new compounds which show an interaction with a particular enzyme. This information provides the possibility to implement the on-line separation/affinity molecule detection process of the present invention in, e.g., drug discovery.

By a "controlled amount" of enzyme and substrate that is added to the effluent is to be understood an amount of known concentration and of known flow rate.

The term "known substrate" as used in the present description and claims refers to a substrate which is capable of interacting or reacting with the enzyme, and which can be detected by the MS.

On-line coupling as used in the methods of the present invention requires fast reaction times in order to minimize extra-column band broadening. This means that interactions having reaction times in the order of minutes rather than hours should be considered. The suitable binding conditions under which the enzymes bind to the analyte comprise a contact time, which is in the same order of magnitude. Suitable binding conditions are conditions that provide optimal binding between the enzyme molecules and the analyte. It will be understood that the precise conditions, such as temperature, residence time, chemical composition, will depend strongly on the type of assay and the proteins used therein. In contrast to biochemical assays based on fluorescent, radioactive or enzyme labels, where background buffer solutions are mainly composed of involatile phosphate or Tris buffers the mass spectrometric assays presented here are performed using volatile buffers such as ammonium formate or ammonium acetate at neutral pH. Moreover, to improve the ionization characteristics of the known ligand, small amounts of methanol (2.5-10%) are added to the background buffer as make-up flow.

The invention will be described in further detail, while referring to the following, non-limiting examples.

### Example 1

The feasibility of the present invention is demonstrated for a protein kinase assay. The kinase catalyzes the phosphorylation of a specific peptide substrate in the presence of ATP. In this example, the following reagents were used:
Enzyme: Protein kinase A (from bovine heart; 80% protein; phosphorylation activity: 1-2 units per µg protein)
Substrate: Malantide (H-Arg-Thr-Lys-Arg-Ser-Gly-Ser-Val-Tyr-Glu-Pro-Leu-Lys-Ile-OH; >97%; FW 1633.9 Da),
Cofactors: Adenosine 3':5'-cyclic monophosphate (free acid; >99%; cAMP), adenosine 5'-triphosphate (magnesium salt; 95-98%; MgATP), P 0300 (H-Thr-Thr-Tyr-Ala-Asp-Phe-Ile-Ala-Ser-Gly-Arg-Thr-Gly-Arg-Asg-Asn-Ala-Ile-His-Asp-OH; FW 2222.4 Da; Inh) were obtained from Sigma (Zwijndrecht, The Netherlands).

### Internal

standard: PP60 c-src (phosphorylated) (H-Thr-Ser-Thr-Glu-Pro-Gln-Tyr(PO₃H₂)-Gln-Pro-Gly-Glu-Asn-Leu-OH; >99%; FW 1543.5 Da; IS2) was supplied by Bachem (Bubendorf, Switzerland).
Inhibitor: P 0300 (H-Thr-Thr-Tyr-Ala-Asp-Phe-Ile-Ala-Ser-Gly-Arg-Thr-Gly-Arg-Arg-Asn-Ala-Ile-His-Asp-OH; FW 2222.4) was obtained from Sigma (Zwijndrecht; The Netherlands).

The carrier flow operated at a flow rate of 10.0 µL min⁻¹ consisted of an aqueous solution containing 20 mM ammonium bicarbonate. Reagent delivery was performed using 10 mL SuperLoops (Pharmacia Biotech AB, Uppsala, Sweden). The input flow of the Superloops was generated using Shicoh Engineering (Yamato, Japan) HPLC pumps connected to the inlet of Superloops by a Peek tubing of 0.5 m length and 0.064 mm inner diameter. The outlet of the Superloops was connected to a reaction detection system comprised of inverted Y-type mixing unions and knitted poly(tetrafluoroethylene) reaction coils with 100 µL or 200 µL internal volume, respectively (i.d. 0.3 mm, length 1.41 and 2.82 m, respectively). The reaction temperature was adjusted to 45°C. Both Superloops were operated at a flow rate of 10.0 µL min⁻¹. Prior to infusion into the mass spectrometer, a make-up solution containing methanol/1% acetic acid was added via an inverted Y-type mixing union at a flow rate of 2.0 µL min⁻¹. The make-up solution was pumped by a syringe pump (Harvard Apparatus 22, Harvard, MA, USA) via syringe (Hamilton, Reno, NV, USA). The total flow was directed via an electrospray interface into the Q-TOF 2 (Micromass, Manchester, UK) mass spectrometer.

In a typical set-up SuperLoop 1 containing the enzyme was filled with 2 mL aqueous solution containing 20 mM ammonium bicarbonate, 100 µM MgSO₄, 1 nM CAMP, 20 µM MgATP, and protein kinase A (224-448 units). The SuperLoop 2 containing the substrate solution was filled with 2 mL aqueous solution containing 20 mM ammonium bicarbonate, 100 µM MgSO₄, 2 µM peptide (malantide). The syringe for the make-up flow was filled with methanol containing 1% acetic acid and 10 µM internal standard.

The MS measurements were carried out on a Q-TOF 2 mass spectrometer coupled to an electrospray interface. The measurements were performed in the positive ionization mode with 353K source temperature, 423K desolvation temperature, 250 L h⁻¹ desolvation gas flow, 100 L h⁻¹ cone gas flow, 16 p.s.i. gas cell pressure, 2500 V capillary voltage, 33 V cone voltage. The mass-range was set to 300 - 1100 m/z and data acquisition parameter were 5.0 sec/scan, 0.1 sec dwell time, full TOF MS continuous scan mode, and without using the option 'MS profile'. Nitrogen (purity 5.0; Praxair, Oevel, Belgium) and argon (purity 5.0; Praxair) were used as desolvation/ cone gas and collision gas, respectively.

In a typical experiment, the mixing of the malantide substrate with Protein kinase A leads to the formation of phosphorylated malantide which can be detected at an m/z value of m/z 571.9. At a reaction temperature of 45°C an injection of a 2 µM solution of the inhibitor leads to a signal change from 3700 counts (arbitrary units) to 1230 counts (arbitrary units) while the counts for the internal standard remain constant indicating that the change of signal of phosphorylated malantide is caused by the deactivation of protein kinase A due to the injection of the inhibitor. A clear influence of the reaction temperature on the assay was observed. At 25°C, an injection of a 2 µM solution of the inhibitor leads to a signal change from 1930 counts (arbitrary units) to 634 counts (arbitrary units) while the counts for the internal standard remain constant.

### Example 2

The same conditions as in example 1 were used, but now kemptide instead of malantide was used as substrate. The mixing of the kemptide substrate with Protein kinase A leads to the formation of phosphorylated kemptide which can be detected at an m/z value of m/z 426.7. At a reaction temperature of 45°C an injection of a 2 µM solution of the inhibitor leads to a signal change from 1240 counts (arbitrary units) to 335 counts (arbitrary units) while the counts for the internal standard remain constant at value of 5030 count. Similar to the phosphorylation of malantide, a clear influence of the reaction temperature on the assay was observed. At 25°C, an injection of a 2 µM solution of the inhibitor leads to a signal change from 1930 counts (arbitrary units) to 820 counts (arbitrary units) while the counts for the internal standard remain constant at value of 5790 counts.

### Example 3

In this example, the use of ESI-TOF-MS (electron spray time-of-flight mass spectrometry) as a detection technique for monitoring enzymatic activity and enzyme inhibition is demonstrated. A homogeneous, substrate-conversion-based bioassay was selected as model system, in which Z-FR-AMC was used as a substrate for cathepsin B. This enzyme, cathepsin B, is a cysteine protease, and belongs to an important group of enzymes involved in many physiological and pathological processes, such as intracellular protein turnover and cancer invasion and metastasis.

The enzymatic assay was performed in a continuous-flow ESI-TOF-MS system where the enzyme cathepsin B and the substrate Z-FR-AMC were subsequently added to the carrier solution of a flow injection system simulating the effluent of an LC column. As a consequence, cathepsin B was continuously incubated with Z-FR-AMC, resulting in a continuous supply of converted substrate. The product was pumped into the mass spectrometer and monitored. Various compounds were injected into the continuous-flow system, incubated with the enzyme in a reaction coil and subsequently pumped into the mass spectrometer. This enabled determination of their inhibition potency as well as characterization of the injected compound. For the screening of extracts, online separation of compounds is required, which was obtained by coupling an LC column in front of the bioassay, resulting in a LC-continuous-flow ESI-TOF-MS system. The ability to screen extracts of natural products is in particularly important for the pharmaceutical industry, as these extracts have often been the starting point for the development of novel drug. Using an online continuous-flow ESI-TOF-MS system, made it possible to screen a mixture for active compounds, demonstrating the applicability of ESI-MS as a valuable approach for primary detection.

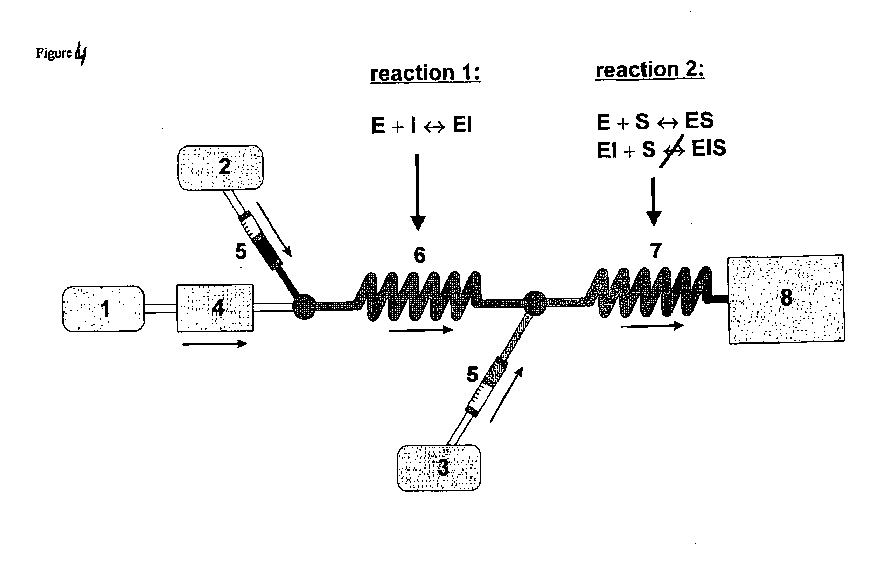

More in detail, samples were analyzed by two different continuous-flow (CF) systems, which both consisted of a homogeneous bioassay coupled to a Micromass (Wythenshawe, UK) Q-Tof micro mass spectrometer. The first continuous-flow system, which is named the FI-CF system (Figure 4), consisted of three Agilent Technologies (Palo Alto, CA, USA) 1100 pumps that delivered the reactants. Superloops (10 mL), manufactured by Pharmacia AB (Uppsala, Sweden), placed after the pumps, were used for the introduction of reagent solutions into the continuous-flow system to prevent nonspecific binding of compounds to pump surfaces, and to reduce the use of expensive chemicals. A Gilson (Middleton, WI, USA) model 234 autoinjector, equipped with a Rheodyne (Cotati, CA, USA) six-port injection valve, was used to inject compounds. Two reaction coils (A, volume of 20 µL; B, volume of 30 µL) consisted of teflon tubing (polytetrafluorethylene, PTFE, 0.25 mm ID) that was knitted to provide radial mixing. Reactants were incubated in both coils at a temperature of 301 K. The enzyme superloop was kept at 285 K using a Spark (Emmen, The Netherlands) Mistral thermostat to minimize enzyme degradation, while the substrate superloop was kept at 301 K (cooling was not necessary). The amount of formed product was determined by MS, which was equipped with a Micromass Z-spray electrospray ionization (ESI) source. Instrument control, data acquisition and data processing were performed using MassLynx software (V4.00.00) software running under Microsoft (Redmond, WA, USA) Windows NT.

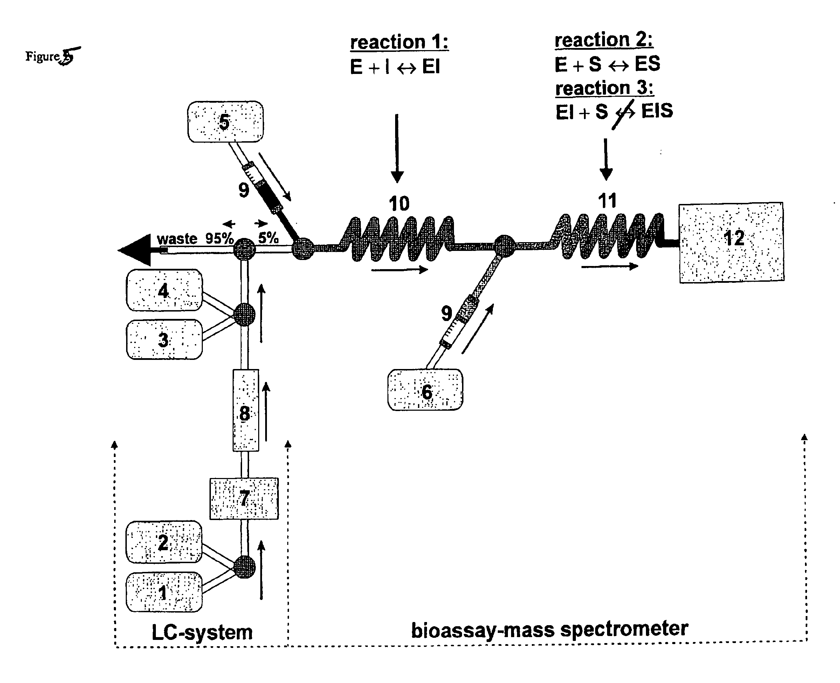

The second continuous-flow system, which is named the LC-CF system, (Figure 5) was largely the same as the FI-CF system. Differences were the replacement of pump 1 and the autoinjector by an LC-system. This LC-system, which is the left part of Figure 5, consisted of two Agilent Technologies 1100 pumps (Figure 5; 6 and 7) delivering a binary gradient. The gradient was pumped through an autoinjector (Gilson, model 235p) equipped with a Rheodyne six-port injection valve, which was temperature controlled (285 K) by a Peltier cooler, and a Zorbax (Agilent Technologies) SB-C18 (5 µM, 2.1 × 150 mm) column, temperature controlled (303 K) by a Shimadzu (Kyoto, Japan) CTO-10AC VP column oven. The column effluent was mixed with a makeup flow, delivered by two Agilent Technologies 1100 pumps (Figure 2; 3 and 4) to decrease the amount of methanol necessary for LC-separation. Gradient and makeup flow were controlled by Kiadis (Leiden, The Netherlands) ScreenControl 2.96h software, running under Microsoft Windows 2000. A Degasys Ultimate from Uniflows (Tokyo, Japan) vacuum degasser was used to degas the solution pumped through the system. To reduce the increased flow rate, a homemade splitter was build in between the LC-system and the bioassay-mass spectrometer, splitting with a ratio of 95% to waste and 5% to the bioassay-mass spectrometer.

ESI-TOF-MS detection was optimized by continuous infusion of a Z-FR-AMC solution by a syringe (Hamilton, Reno, NV, USA) and a kdScientific (New Hope, PA, USA) syringe pump (model KSD100) at a flow rate of 10 µL/min. Best settings for sensitivity for Z-FR-AMC were 3300 V capillary, 30 V sample cone, 3 V extraction cone, 448 K desolvation temperature, 353 K source temperature and a scan range of m/z 150-650. Measurements were performed in positive ionization mode combined with a cone gas flow of 100-200 L/hr and a desolvation gas flow of 400-600 L/hr. Nitrogen (99.999% purity) was used as gas.

Cathepsin B (E.C. 3.4.22.1, from bovine spleen, activity: 19 units/mg protein, unit definition: one unit will hydrolyze 1 micromole of N-α-CBZ-lysine P-nitrophenyl ester per min at pH 5.0 at 298 K), antipain (N-(Nα-carbonyl-Arg-Val-Arg-al)-Phe, *M*ᵣ 604.7), E64 (trans-epoxysuccinyl-L-leucylamido-(4-guanidino)butane, *M*ᵣ 357.4), cAMP (internal standard 1, adenosine 3',5'-cyclic monophosphate, *M*ᵣ 329.2), biotin (internal standard 2, *M*ᵣ 244.3) and DTE (1,4-dithioerythritol, *M*ᵣ 154.3) were purchased from Sigma (St. Louis, MO, USA). CA-074 (L-trans-epoxysuccinyl-Ile-Pro-OH propylamide, *M*ᵣ 383.5), Z-FR-AMC (CBZ-Phe-Arg 7-amido-4-methylcoumarin hydrochloride, *M*ᵣ 649.2), ovokinin (Phe-Arg-Ala-Asp-His-Pro-Phe-Leu, *M*ᵣ 1002.1) and pp60 c-src (phosphorylated, Thr-Ser-Thr-Glu-Pro-Gln-Tyr(PO₃H₂)-Gln-Pro-Gly-Glu-Asn-Leu, *M*ᵣ 1543.5) were obtained from Bachem (Bubendorf, Switzerland). Methanol and ammonium hydroxide were from J. T. Baker (Deventer, The Netherlands), acetic acid from Riedel-de Haën (Seelze, Germany), ammonium formate from Aldrich (Steinheim, Germany) and high purity water was from a Milli-Q system (Bedford, MA, USA).

Reactants and internal standards were dissolved in a carrier fluid, which consisted of 20 mM ammonium formate dissolved in water, set at pH 7.0 by ammonium hydroxide. DTE was added to the carrier to increase enzyme activity at a concentration of 15.0 µM and 22.5 for the FI-CF system and LC-CF system, respectively. DTE was not present in solutions that were pumped through the LC column to minimize disturbance of the separation, and not present in the makeup flow solution.

Batches containing 20 mM ammonium formate, various concentrations of ovokinin (0.10 - 2.0 µM) and pp60 c-src (0.20 - 4.0 µM), 10% (v/v) methanol and 0.1% (v/v) acetic acid were prepared, resulting in an aqueous solution of pH 4.3. Samples prepared were infused into the mass spectrometer by a syringe pump (kdScientific) at a flow rate of 10 µL/min.

For the flow-injection-analysis, solutions were prepared as described for the reactants and internal standards and put in superloops. Used concentrations were 30 nM cathepsin B and 6 µM internal standard 1, and 50 µM Z-FR-AMC and 6 µM internal standard 2, for superloop 1 and superloop 2, respectively.

Two inhibitors were selected, E64 and antipain, which were injected (1 µL) in duplicate at various concentrations (E64: 0.50, 1.0 and 2.5 µM; antipain: 0.10, 0.25 and 0.50 µM).

Batches containing 20 mM ammonium formate (pH 7.0), various amounts of Z-FR-AMC (0, 0.50, 1.0, 2.5, 5.0 and 7.5 µM), 7.5 nM cathepsin B, 1.5 µM internal standard 1, 1.5 µM internal standard 2 in water were prepared. ESI-TOF-MS analysis of the batches was performed, after 60 min incubation at 301 K, by infusion using a syringe pump at a flow rate of 10 µL/min.

Batches containing 20 mM ammonium formate, and various concentrations of cAMP and biotin (0.50 - 5.0 µM) were prepared, resulting in an aqueous solution of pH 4.3. ESI-TOF-MS analysis of the batches was performed by infusion using a syringe pump at a flow rate of 10 µL/min.

For the HPLC screening, solutions were prepared as described herein-above and filled in superloops. Used concentrations were 30 nM cathepsin B and 6 µM internal standard 1, and 50 µM Z-FR-AMC and 6 µM internal standard 2, for superloop 1 and superloop 2, respectively. Injected inhibitors: E64 and CA074, which were in mixture injected (E64: 40 µM; CA-074: 90 µM). The chromatographic conditions for gradient elution were: flow rate, 200 µL/min; volume injected, 20 µL, column temperature, 298 K; and autoinjector temperature, 283 K. The mobile phase was a binary gradient mixture of 20 mM ammonium formate in water at pH 7.0 and methanol. The gradient started at 30% methanol (v/v) which was increased to 35% in 12 min.

### Assay setup.

A homogeneous bioassay was coupled to a mass spectrometer to form together the FI-CF system, as shown in Figure 4. Carrier solution (20 µL/min) was mixed continuously with cathepsin B solution (10 µL/min) in reaction coil A for 40 sec, and carrier/cathepsin B solution with Z-FR-AMC solution (30 µL/min) in reaction coil B for 45 sec. The resulting flow (40 µL/min) coming out of reaction coil B was pumped into the mass spectrometer, where the amount of formed product was directly monitored by ESI-TOF-MS. Compounds injected via the autoinjector into the carrier flow were allowed to react with cathepsin B for 40 sec (Figure 4, reaction 1). After mixing in reaction coil A, both inhibitor and enzyme were pumped into reaction coil B, where they were mixed with the substrate. Without inhibitors, a continuous cleavage of Z-FR-AMC took place in the first reaction coil. However, if an inhibitor blocked the enzyme in the first reaction coil, a temporary decrease of cleaved substrate occurred (Figure 4, reactions 2 and 3).

The second system used was the LC-CF system, as shown in Figure 5, which was largely the same as the FI-CF system. Compounds injected via an autoinjector were separated by the LC-column, using a binary gradient system consisting of carrier solution and methanol. In order to reduce the amount of methanol that was necessary for separation, a makeup solution was added to the column effluent, resulting in a constant methanol concentration of 10%. Decreasing the concentration of methanol was necessary to prevent enzyme degradation. The flow rate over the column was 200 µL/min, which was increased to 960 µL/min after addition of the makeup flow. This flow (960 µL/min) was splitted with a ratio of 95% to waste and 5% (48 µL/min) to the bioassay-mass spectrometer. From this point, the FI-CF system was more or less the same as the LC-CF system, only the flow rates differed. In the LC-CF system, both the enzyme solution and the substrate solution were delivered at a flow rate of 50 µL/min, resulting in a total flow rate of 100 µL/min into the mass spectrometer. As a consequence, the reaction times were decreased to 16 and 18 sec for reaction coil A and B, respectively. The reduced reaction times resulted in a lower amount of product, but still enough for a good performance of the LC-CF system.

The combination of bioassays and MS as used in this continuous-flow system requires a solvent composition compatible to both. Enzymatic assays are mostly performed in solutions containing nonvolatile buffer salts such as HEPES, TRIS and PBS, and additives that preserve enzyme activity and nonspecific binding to surfaces. However, nonvolatile salts contaminate the ion source of the mass spectrometer and decrease the MS performance. Moreover, the amount of additives has to be kept as low as possible, as they cause signal suppression in ESI. For this reason, commonly used additives and nonvolatile buffer salts were omitted in this work. A volatile salt (ammonium formate) was selected, which is known to be suitable for both the bioassay and ESI-TOF-MS. The carrier solution contained only DTE, which is necessary for cathepsin B activation. Furthermore, in many mass spectrometric experiments an organic modifier is added to increase the solubility of the analytes and to promote the desolvation of the charged droplets formed by the electrospray process. In addition, lower pH values will also increase the mass spectrometric performance, as positive ions (detected in the positive ion mode of the mass spectrometer) are more readily formed in acidic solutions. In many cases, however, both organic modifier and an acidic pH, however, are not compatible with enzymatic reactions. For this reason, a neutral pH (7.0) and a bioassay compatible concentration of organic modifier in the carrier solution were selected.

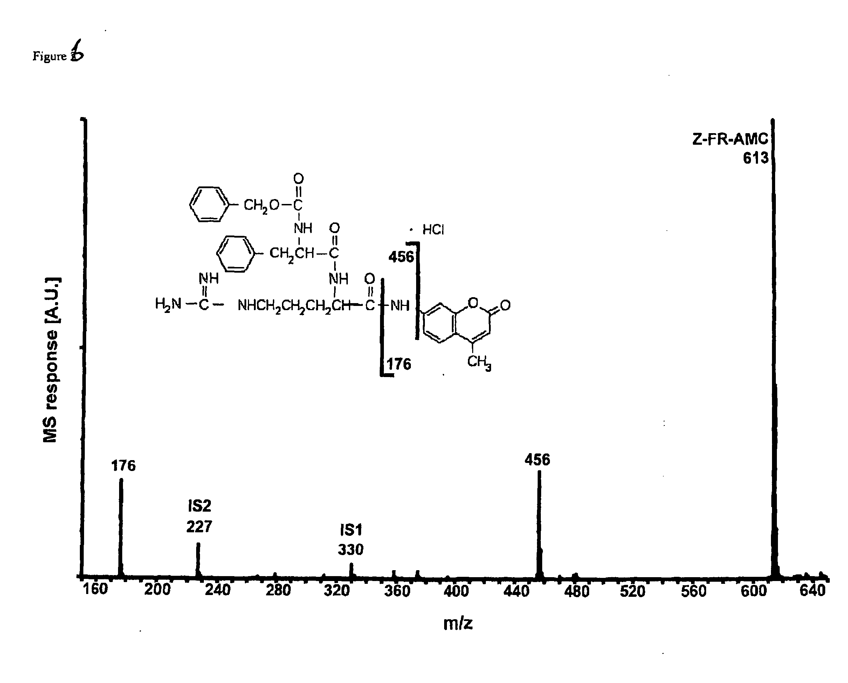

Z-FR-AMC, a fluorescent labeled compound, was selected as substrate for cathepsin B. Figure 6 is a spectrum of Z-FR-AMC after cleavage by cathepsin B, monitored by ESI-TOF-MS. The spectrum shows that enzymatic cleavage of Z-FR-AMC resulted in two products, namely m/z 176 and m/z 456. Furthermore, it shows the uncleaved substrate (m/z 613; without hydrochloride) and two internal standards (biotin, m/z 227, protonated molecule minus water; cAMP, m/z 330). Important information about the sensitivity of the system (internal standards), and the amount of substrate and formed product were easily determined. Two internal standards were used, one in each superloop, to control the flow rates by following the ratio between their intensities.

The precision of the ESI-TOF-MS, expressed as RSD, was studied using five different concentrations of the compounds ovokinin and phosphorylated pp60 c-src. These compounds were selected for the reason that for both ESI-TOF-MS information was present, as they were used in previous studies.

The inter-day (n = 9) precision measurements was 5.1% and 5.5% for ovokinin and pp60 c-src, respectively. The ratio between both compounds was also controlled and fluctuated by 4.8% (RSD). These percentages demonstrate the stability of the MS detection system. However, fluctuations in sensitivity can easily be corrected for the use of an internal standard.

Flow-injection analysis was performed to determine the inhibition potency of two compounds, using the FI-CF system. Two inhibitors were selected, E64 and antipain, which are nonselective serine and cysteine protease inhibitors. Both inhibitors were injected via the autoinjector into the FI-CF system. If the inhibitors block the cathepsin B, negative peaks should show up, as a consequence of temporary decreased amount of active enzyme.

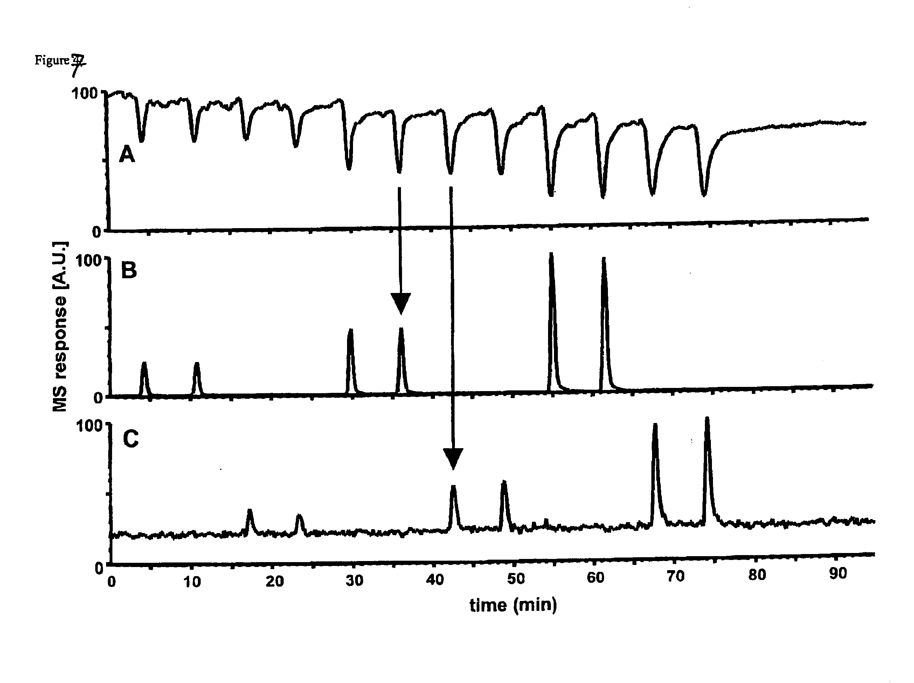

The result of this flow-injection analysis is depicted in Figure 7. All three mass traces are extracted ion chromatograms (EIC), in which an ion of interest is extracted out of the total amount of ions detected. The first mass trace is an EIC of m/z 456.0 (formed product), the second mass trace of m/z 190.7 (fragment of antipain), and the third mass trace of m/z 358.2 (E64).

Inhibitor injections resulted in negative peaks in the product mass trace. These negative peaks could be caused by components that largely suppressed the ion formation, or by components that inhibited the enzyme resulting in a lower amount of product. The first option is controlled by the usage of internal standards. To be sure that the negative peaks were not due to selective suppression, the mass traces of both products (m/z 176 and m/z 456) were compared, showing that they did not differ. In addition, blank injections and injections of cAMP (10 µM) did not result in negative peaks (data not shown). This is further confirmed by the linearity of the calibration curves (see Quantification). If the products were sensitive for signal suppression, linear curves for both masses (R² = 0.9964 and R² = 0.9972 for m/z 176 and m/z 456, respectively) would not have been obtained. If a compound was suppressing the ionization process, a similar decrease in ion current should be observed for the internal standards (or other compounds). However, as the intensity of the internal standards remained unchanged during the negative peaks (data not shown), signal suppression could be ruled out. To determine if the second option was true, background ions were subtracted from the negative peaks (by using the Micromass software), resulting in spectra that belonged to the inhibitors. This fact in combination with the knowledge that the negative peaks were not a result of ion suppression, lead to the conclusion that the injected inhibitors blocked the enzyme in the first reaction coil. Determination of the molecular mass of the inhibitors was possible as only a part of the total amount of inhibitory compounds was bound to cathepsin B. However, obtaining useful mass spectra is only possible if the inhibitor is ionizable.

Besides the determination of which component has blocked the inhibitor, it is also possible to determine the binding strength of an inhibitor. The area of the negative peaks is an indication of the inhibition potency of the compounds, in other words, the stronger the inhibitor, the larger the area of the negative peak. This is not possible for natural product extracts, where the concentrations of the inhibitory compounds are unknown. Considering Figure 7, it is obvious that antipain is a stronger inhibitor than E64, as the area of peaks caused by 0.10 µM antipain are more or less the same as the area of the peaks caused by 0.50 µM E64. It is also very clear that higher concentrations inhibitor block larger amounts of enzyme, resulting in larger negative peaks.

Looking at the EIC of a product (top mass trace), one can see that the MS intensity is decreasing against the time. This is a consequence of nonspecific binding of the enzyme to the tubing, as the same effect was not observed for the internal standards.

Summarizing, affinity constants of extracted or synthesized compounds can be determined with this system. The usage of internal standards made it possible to control signal suppression. Limits of detection were in the order of 50-250 nM, which was in agreement with fluorescence detection, where the detection limits were in the order of 25-100 nM (data not shown), demonstrating the suitability of the ESI-TOF-MS mode.

An important factor is the quantification of the formed product, which makes it possible to indicate the strength of the inhibitor. Quantification was started by preparing batches containing the necessary reactants and internal standards and various concentrations of Z-FR-AMC. Batches were incubated for 60 min to be sure that all Z-FR-AMC was cleaved by cathepsin B. ESI-TOF-MS analysis, performed directly after incubation, was done by infusion (kdScientific syringe pump) of the samples into the mass spectrometer. After controlling that all Z-FR-AMC was cleaved, the amount of formed product was determined by averaging the acquired spectra. Signal intensities obtained were corrected for signal suppression, and plotted (data not shown) against the concentration Z-FR-AMC, creating linear graphs for m/z 176 (product 1; R² = 0.9964) and m/z 456 (product 2; R² = 0.9972). Out of this plot, the concentration product formed could be obtained for each analysis by interpolation. As a negative peak showed up, the product intensity decreased, resulting in a lower concentration calculated by interpolation. Differences in concentration can be calculated, and compared with other peaks to get a indication of the affinity of the injected compound for the enzyme. For real quantitative data, more measurements should be performed.

Differences in signal suppression or MS sensitivity can be controlled by the use of internal standards. In this research, calibration curves of the internal standards cAMP (R² = 0.9935) and biotin (R² = 0.9962) were obtained by continuous infusion into the mass spectrometer.

The RP-separation was performed by a binary gradient system, using water and methanol. The organic modifier, however, decreases the activity of enzymes. For this reason, a makeup flow was added after the LC-column (see Figure 5) to reduce the concentration of methanol. This resulted in an increased post column flow rate (960 µL/min) and a lower analyte concentration due to dilution. To reduce the flow rate, which was too high for the bioassay and mass spectrometer, the flow was reduced to 48 µL/min by placing a flow splitter between the LC-column and the bioassay. This flow was used as the carrier solvent that was pumped into the continuous-flow system.

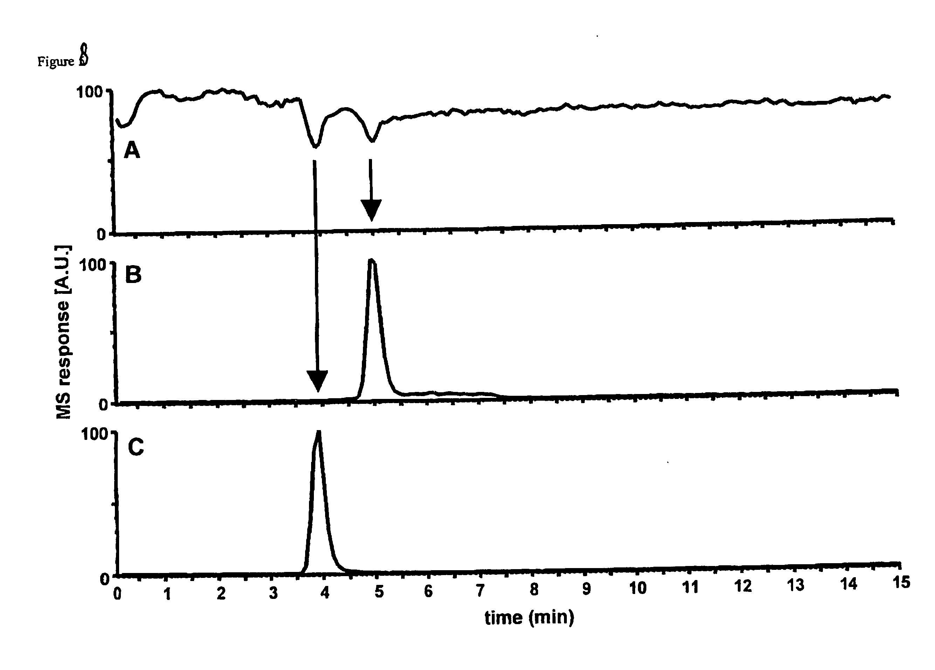

The described LC-CF system was used for the determination of the inhibition potency of the compounds E64 and CA-074. ESI-TOF-MS was used for this analysis, following the amount of formed product by an EIC (Figure 8; A). During the first three minutes, the product mass trace was stable (controlled by internal standards). At 3.8 and 4.8 min, negative peaks showed up in the product mass trace. As mentioned earlier, this could be a consequence of signal suppression by eluted components or a consequence of cathepsin B inhibition. Internal standards mass traces showed that the negative peaks were caused by inhibition. The next step was the determination of the compounds that had bound to the enzyme, by background subtraction using Micromass software. Extracted m/z values were 358.2 and 384.2 for peak 1 and peak 2, respectively, corresponding to the protonated molecules of the compounds E64 (Figure 8, C) and CA-074 (Figure 8; B). Concluding this, injected analytes were separated by the LC-column, mixed well in the bioassay with the enzyme and subsequently pumped into the mass spectrometer. This assay is not limited to a mixture containing two components, but also for more complex mixtures, provided that they are separated. For this reason, the online continuous-flow screening assay based on ESI-TOF-MS is a good alternative detection technique to fluorescence- and radioactivity-based detection methods for primary screening of extracts and other mixtures.

## Claims

1. On-line detection method comprising the steps of: contacting an effluent of a fractionation step with a controlled amount of an enzyme; allowing the enzyme to interact with compounds capable of binding to or intimately interacting with said enzyme so that the enzyme properties are affected, said compound being suspected to be present in the effluent; addition of a controlled amount of a substrate for said enzyme; allowing a reaction of the enzyme with the substrate providing one or more modified substrate products; and detection of unreacted substrate, or a modified substrate product using a mass spectrometer.

2. The method according to claim 1, involving detection of a modified substrate product.

3. The method according to claim 2, wherein the reaction mixture passes a hollow-fibre module, separating off molecules which have a higher molecular weight said modified substrate product, prior to entering the mass spectrometer.

4. The method according to any one of the preceding claims, wherein the detection is based on specific m/z values for the substrate or the modified substrate product.

5. The method according to any one of the preceding claims, using electrospray ionisation mass spectrometry.

6. The method according to any one of the preceding claims, wherein a make-up flow is added to the reaction mixture resulting from the reaction with the substrate, prior to the introduction in the mass spectrometer.

7. The method according to any one of the preceding claims, wherein said fractionation step is a liquid chromatography separation, a capillary electrophoresis step or a combinatorial chemistry system.

8. The method according to any one of the preceding claims, wherein the liquid chromatography separation step is an HPLC, a reversed phase HPLC, a CE, a CEC, an IEF or an MEKC step.

9. The method according to any one of the preceding claims, using a mass spectrometer selected from the group consisting of electrospray ionization type, atmospheric pressure ionization type, quadrupole type, magnetic sector type, time-off-flight type, MS/MS, MSⁿ, FTMS type, ion trap type and combinations thereof.

10. The method according to any one of the preceding claims, wherein said enzyme is a mixture of two or more different types of enzymes and said substrate is present in a mixture of different substrates, each of which substrate being specific for one of said enzymes.

11. The method according to any one of the preceding claims, wherein the enzyme or one of the enzymes is a protein kinase, and wherein the detection is carried out on a phosphorylated product of a kinase catalysed reaction.

12. On-line detection method according to claim 1, wherein a mass spectrometer with a multiple-inlet unit is used, to which multiple-inlet unit different fractionation lines are connected, wherein each fractionation line comprises an effluent to which controlled amounts of enzyme and known substrates are added as described in each of the previous claims.

## Patentansprüche

1. Online Nachweisverfahren umfassend die Schritte: Kontaktieren einer Austrittströmung eines Fraktionierungsschritts mit einer kontrollierten Menge an einem Enzym; Erlauben dem Enzym, mit zum Binden an oder zur engen Wechselwirkung mit dem Enzym befähigten Verbindungen zu interagieren, so dass die Enzymeigenschaften beeinflusst werden, wobei die Verbindung in der Austrittströmung vorliegt; Zugabe einer kontrollierten Menge eines Substrats für das Enzym; Erlauben einer Reaktion des Enzyms mit dem Substrat liefernd ein oder mehr modifizierte Substratprodukte und Nachweis des unreagierten Substrats oder eines modifizierten Substratprodukts unter Einsatz eines Massenspektrometers.

2. Verfahren nach Anspruch 1, welches den Nachweis eines modifizierten Substratprodukts umfasst.

3. Verfahren nach Anspruch 2, wobei die Reaktionsmischung ein Hohlfaser-Modul passiert, um vor dem Einströmen in den Massenspektrometer Moleküle, welche ein höheres Molekulargewicht als das modifizierte Substratprodukt aufweisen, abzutrennen.

4. Verfahren nach einem der vorgehenden Ansprüche, wobei der Nachweis auf spezifischen m/z-Werten für das Substrat oder für das modifizierte Substratprodukt basiert.

5. Verfahren nach einem der vorhergehenden Ansprüche unter Einsatz von Elektrospray-Ionisations-Massenspektometrie.

6. Verfahren nach einem der vorhergehenden Ansprüche, wobei der Reaktionsmischung ein aus der Reaktion mit dem Substrat vor der Einführung in den Massenspektrometer resultierender Zusatzstrom zugefügt wird.

7. Verfahren nach einem der vorhergehenden Ansprüche, wobei der Fraktionierungsschritt eine Flüssigchromatographietrennung, ein Kapillarelektrophoreseschritt oder ein kombinatorisches Chemiesystem ist.

8. Verfahren nach einem der vorhergehenden Ansprüche, wobei der Flüssigchromatographietrennschritt eine HPLC, eine Umkehrphasen HPLC, eine CE, eine CEC, eine IEF oder ein MEKC-Schritt ist.

9. Verfahren nach einem der vorhergehenden Ansprüche unter Einsatz eines Massenspektrometers ausgewählt aus der Gruppe bestehend aus solchen vom Elektrospray-Ionisations-Typ, Ionisations-Typ bei atmosphärischem Druck, Quadrupol-Typ, Magnetsektor-Typ, Flugzeit-Typ, MS/MS, MSⁿ, FTMS-Typ, Ionenfallen-Typ und Kombinationen hiervon.

10. Verfahren nach einem der vorhergehenden Ansprüche, wobei das Enzym eine Mischung aus zwei oder mehr verschiedenen Arten an Enzymen ist und das Substrat in einer Mischung von verschiedenen Substraten vorliegt, wobei jedes dieser Substrate für eines der Enzyme spezifisch ist.

11. Verfahren nach einem der vorhergehenden Ansprüche, wobei das Enzym oder eines der Enzyme eine Protein-Kinase ist, und, wobei der Nachweis auf einem phosphoryliertem Produkt einer kinasekatalysierten Reaktion durchgeführt wird.

12. Online Nachweisverfahren nach Anspruch 1, wobei ein Massenspektromer mit einer Vielfacheinlasseinheit eingesetzt wird, wobei mit der Vielfacheinlasseinheit unterschiedliche Fraktionierungsleitungen verbunden werden, wobei jede Fraktionierungsleitung einen Austritt umfasst, zu dem wie in jedem der vorhergehenden Ansprüche beschrieben kontrollierte Mengen an Enzym und bekannten Substraten zugefügt werden.

## Revendications

1. Procédé de détection en ligne comprenant les étapes consistant : à mettre en contact un effluent d'une étape de fractionnement avec une quantité régulée d'une enzyme ; à laisser l'enzyme interagir avec des composés aptes à se lier à ou à interagir intimement avec ladite enzyme de sorte que les propriétés de l'enzyme sont affectées, ledit composé étant suspecté d'être présent dans l'effluent ; à ajouter une quantité régulée d'un substrat pour ladite enzyme ; à permettre une réaction de l'enzyme avec le substrat, fournissant un ou plusieurs produits de substrat modifiés ; et à détecter le substrat n'ayant pas réagi, ou un produit de substrat modifié en utilisant un spectromètre de masse.

2. Procédé selon la revendication 1, comprenant la détection d'un produit de substrat modifié.

3. Procédé selon la revendication 2, dans lequel le mélange réactionnel passe dans un module à fibres creuses, séparant les molécules qui ont un poids moléculaire plus élevé que ledit produit de substrat modifié, avant d'entrer dans le spectromètre de masse.

4. Procédé selon l'une quelconque des revendications précédentes, dans lequel la détection repose sur des valeurs m/z spécifiques pour le substrat ou le produit de substrat modifié.

5. Procédé selon l'une quelconque des revendications précédentes, utilisant une spectrométrie de masse à ionisation par électrospray.

6. Procédé selon l'une quelconque des revendications précédentes, dans lequel un flux de complément est ajouté au mélange réactionnel résultant de la réaction avec le substrat, avant l'introduction dans le spectromètre de masse.

7. Procédé selon l'une quelconque des revendications précédentes, dans lequel ladite étape de fractionnement est une séparation par chromatographie liquide, une étape d'électrophorèse capillaire ou un système de chimie combinatoire.

8. Procédé selon l'une quelconque des revendications précédentes, dans lequel l'étape de séparation par chromatographie liquide est une étape de CLHP, de CLHP en phase inverse, d'électrophorèse capillaire (EC), d'électrochromatographie capillaire (CEC), de focalisation isoélectrique (IEF) ou de chromatographie électrocinétique micellaire (MEKC).

9. Procédé selon l'une quelconque des revendications précédentes, utilisant un spectromètre de masse choisi dans le groupe constitué par le type ionisation par électrospray, le type ionisation à pression atmosphérique, le type quadrupôle, le type à secteur magnétique, le type à temps de vol, MS/MS, MSⁿ, le type FTMS, le type à piège ionique et des combinaisons de celles-ci.

10. Procédé selon l'une quelconque des revendications précédentes, dans lequel ladite enzyme est un mélange de deux ou plusieurs sortes différentes d'enzymes et ledit substrat est présent dans un mélange de différents substrats, dont chaque substrat est spécifique pour l'une desdites enzymes.

11. Procédé selon l'une quelconque des revendications précédentes, dans lequel l'enzyme ou l'une des enzymes est une protéine kinase, et dans lequel la détection est effectuée sur un produit phosphorylé d'une réaction catalysée par kinase.

12. Procédé de détection en ligne selon la revendication 1, dans lequel un spectromètre de masse avec un dispositif à multiples entrées est utilisé, dispositif à multiples entrées auquel différentes conduites de fractionnement sont raccordées, dans lequel chaque conduite de fractionnement comprend un effluent auquel des quantités régulées d'enzyme et de substrats connus sont ajoutées comme décrit dans chacune des revendications précédentes.
